# FASCICULE DE BREVET EUROPEEN

(11) **EP 1 174 178 B1**
(45) Date de publication et mention de la délivrance du brevet: **03.05.2006**
(21) Numéro de dépôt: 01401872.5
(22) Date de dépôt: 12.07.2001
(51) Int. Cl.: B01F 3/02, B01F 15/04, A61M 16/12, G05D 11/13

(54) **Procédé et installation de conditionnement en dynamique de gaz notamment à usage medical**
Verfahren und Anlage zur dynamischen Behandlung von Gas, insbesondere für medizinische Zwecke
Process and plant for dynamic gas conditioning, in particular for medical use

(30) Priorité: 18.07.2000 FR 0009412
(43) Date de publication de la demande: 23.01.2002
(73) Titulaire: Air Liquide Santé (International), 75341 Paris Cedex 07 (FR); Air Liquide Santé France, 75341 Paris Cedex 07 (FR)
(72) Inventeur: Wagenheim, Serge, 78500 Sartrouville (FR)
(74) Mandataire: Pittis, Olivier

(56) Documents cités:
- EP-A- 0 566 488
- FR-A- 2 532 858
- FR-A- 2 548 549
- GB-A- 1 382 338
- US-A- 4 345 612
- US-A- 5 636 626

## Description

L'invention concerne une installation de fabrication et de conditionnement en dynamique de mélanges de gaz médicaux, en particulier de mélanges gazeux N₂O/O₂ utilisables dans le domaine médical, notamment en analgésie, de préférence des mélanges N₂O/O₂ contenant approximativement 50% en vol. de protoxyde d'azote (N₂O) et 50% vol. d'oxygène.

Il existe actuellement, au plan industriel, différentes méthodes ou procédés de fabrication et de conditionnement de mélanges gazeux.

Toutefois, dans celles-ci, les quantités de gaz à mélanger sont introduites dans un mélangeur de gaz, sont contrôlées par mesure de la pression et de la température des gaz. Le comptage est donc basé sur deux instruments de mesures qui additionnent leurs imprécisions de mesure, pouvant ainsi conduire à des résultats assez aléatoires.

Par ailleurs, le choix des points de mesure sur l'installation ne permet pas ou incomplètement d'accéder aux grandeurs physiques recherchées et donc de réaliser efficacement et de manière fiable le mélange.

Ainsi, la température est habituellement mesurée sur la rampe de conditionnement du gaz par une sonde de température qui ne reflète pas ou que très imprécisément la température effective des gaz à l'intérieur des emballages de conditionnement.

Parfois, cette mesure s'opère directement sur la surface de l'emballage (bouteille) par une sonde thermique infrarouge ; on comprend que cette mesure n'est pas l'exact reflet de celle du gaz dans l'emballage.

Par ailleurs, on utilise un capteur de pression pour mesurer en permanence la pression sur les rampes de conditionnement, les gaz s'écoulant alors dans les tuyauteries.

Il existe donc, de ce fait, un écart inévitable entre la pression finale dans les emballages, en fin de conditionnement, et la pression mesurée en cours de remplissage, lequel est fonction des pertes de charge, du débit et de la température des gaz.

La méconnaissance des coefficients de perte de charge et de la température exactes des gaz oblige donc à contrôler la pression en statique, en fin de cycle d'injection, c'est-à-dire a posteriori.

De là, si les quantités de gaz mélangées sont non conformes, il convient alors de compléter les quantités de gaz mélangées en rajoutant la quantité de gaz manquante, ce qui n'est pas pratique ou toujours aisément réalisable.

Cependant, à l'inverse, tout excès de gaz fausse complètement la justesse du mélange gazeux désiré et engendre soit la mise au rebut du mélange gazeux ainsi obtenu ou nécessite un réajustement pour tenter de rétablir l'équilibre, ce qui n'est pas toujours possible.

Par ailleurs, on connaît aussi un procédé de conditionnement de mélanges gazeux à base de dioxyde de carbone (CO₂), appelé procédé de conditionnement en dynamique, dans lequel le CO₂ est conditionné à partir d'un état supercritique à 270 bars de pression et à une température entre environ 70°C et 120°C, la pression et la température du gaz étant déterminées par les conditions de fonctionnement du procédé de conditionnement.

Les pressions des différentes sources gaz doivent être équilibrées à 270 bars car la pression est définie de telle sorte que des récipients à 200 bars de pression puissent être conditionnés même en été lorsqu'ils sont chauds car habituellement stockés à l'extérieur, ce qui implique que la source de remplissage en aval de la chambre de mélange doit donc pouvoir atteindre 240 bars (pour un récipient à une température allant jusqu'à 60°C).

De plus, les pertes de charge d'un tel mélangeur dynamique atteignent souvent 20 bars et dès lors les pressions des sources de gaz doivent donc atteindre 260 bars au minimum.

Jusqu'à présent, le CO₂ est le seul gaz liquéfié ayant déjà été conditionné en dynamique.

Lors d'un cycle de conditionnement, les sources de gaz sont détendues en aval de la chambre de mélange jusqu'à la pression des récipients et la pression en aval de la chambre varie, lors du cycle, de quelques mbars à la pression finale de conditionnement du mélange gazeux.

Dans le cas du CO₂ à une pression de 270 bars, la température est de 70°C et est choisie de telle sorte que la détente ne s'accompagne pas d'un changement d'état du CO₂ avec passage à l'état solide (neige carbonique), surtout lorsque la pression est inférieure à 5 bars.

En effet, toute formation de neige carbonique risque d'obstruer les robinets des bouteilles induisant de la sorte des disparités dans les teneurs des mélanges gazeux réalisées dans les bouteilles d'une même rampe de conditionnement.

De ce fait, seuls des mélanges gazeux contenant une teneur en CO₂ ne dépassant en général pas 30% peuvent être produits car sinon la température atteinte en aval de la chambre de détente serait inférieure à la température de dé-mélange.

Les mélanges gazeux dont la teneur en un composé donné est supérieure à 30% (en vol.) sont, quant à eux, habituellement réalisés via des méthodes de fabrication plus classiques, par exemple par gravimétrie avec contrôle des masses introduites dans les bouteilles par pesée ou par mesure de pression corrigée en température. Cependant, ces méthodes présentent les inconvénients de rendre quasi-obligatoire un roulage des bouteilles après mélange pour en homogénéiser le contenu et un contrôle analytique des récipients pour s'assurer de leur conformité aux spécifications visées. Ces façons de procéder ne sont donc pas très pratiques et coûteuses en temps et en productivité.

En outre, pendant un mélange en dynamique se pose aussi le problème de la démixtion du mélange gazeux en aval de la chambre de mélange, c'est-à-dire un démélange ou une séparation intempestive des différents composés du mélange en aval du site où s'effectue ledit mélange.

Le démélange ou démixtion d'un mélange gazeux se caractérise par la séparation en deux phases distinctes dudit mélange, à savoir une phase gazeuse et une phase liquide.

La démixtion se produit dès que la température du mélange s'abaisse en dessous d'un seuil de température. Plus la teneur en gaz du mélange est élevée plus la température de démélange est élevée.

Pour un mélange gazeux binaire formé de 50% de O₂ et 50% de N₂O, ce seuil de démélange se situe à environ -5,5°C, comme expliqué par le document "*Equilibria for mixtures of oxygen with nitrous oxide and carbon dioxide and their relevance to the storage of N2O*/*O2 cylinders for use in analgesia*", mars 1970.

Or, le conditionnement de gaz par un mélangeur dynamique s'accompagne toujours d'une détente en aval de la chambre de mélange et donc en général d'un abaissement de la température des gaz, voire en dessous de la température de démélange dans le cas d'un mélange analgésique.

La circulation des gaz est alors diphasique dans les rampes de conditionnement vers les bouteilles ; la phase liquide et la phase gaz se déplaçant à des vitesses d'écoulement différentes.

Dès lors, le remplissage des bouteilles n'est plus homogène et on constate que des écarts plus ou moins importants de teneurs finales des mélanges réalisés dans chacune des bouteilles conditionnées sur la même rampe lors d'un même cycle ou processus de fabrication.

Ces disparités s'expliquent par des écoulements préférentiels de certains constituants du mélange gazeux par rapport à d'autres dans les tuyauteries des rampes de remplissage des emballages, à savoir des écoulements gravitaires ou en gouttelettes dans le cas des gaz liquéfiés.

Ainsi, dans des conditions de débit de remplissage élevées ou dans le cas d'emballages de faible volume de type B5 (5 litres), les teneurs de mélanges réalisées dans quelques emballages d'un même lot de fabrication, peuvent être en dehors des tolérances de réalisation imposées par la pharmacopée, à savoir un écart maximal de 1% pour un mélange O₂/N₂O à 50%/50% en volume. Dès lors, le contrôle par analyse de chaque emballage est indispensable, ce qui est fastidieux et peu pratique au plan industriel.

Par ailleurs, le document EP-A-566 488 décrit un procédé et dispositif d'élaboration de mélanges gazeux anesthésiques utilisables en anesthésie par inhalation. D'après ce document, l'agent anesthésique est prélevé sous forme liquide, vaporisé puis introduit dans un mélangeur où il est mélangé avec un mélange pré-établi d'oxygène et d'un autre gaz vecteur.

De là, le but de la présente invention est de pouvoir réaliser des mélanges gazeux, en particulier des mélanges gazeux destinés au domaine médical, et de les conditionner ensuite de façon rapide, sûre et efficace, c'est-à-dire sans rencontrer les problèmes se posant avec les procédés dé conditionnement classiques.

Dit autrement, le problème qui se pose est de pouvoir réaliser et conditionner en dynamique des gaz pour réaliser des mélanges gazeux contenant principalement un ou plusieurs des constituants suivants O₂, CO₂, N₂, He et N₂O dans des proportions prédéfinies, de manière à permettre de réaliser des mélanges ayant une teneur variable en un gaz donné, notamment du CO₂ et du N₂O en teneurs supérieures à 30% en particulier un procédé utilisable pour réaliser des mélanges gazeux médicaux, par exemple des mélanges gazeux binaires équivolumiques 50%/50% analgésiques oxygène/protoxyde d'azote, tout en évitant d'atteindre le point ou seuil de température de démélange du mélange gazeux ainsi réalisé.

En d'autres termes, le procédé de l'invention doit présenter les avantages du procédé de conditionnement dynamique du CO₂ connu sans en avoir les inconvénients, c'est-à-dire pouvoir être utilisé pour fabriquer des mélanges gazeux ayant en particulier une teneur de plus de 30% en un gaz donné, tel le protoxyde d'azote (N₂O) ou l'oxygène, tout en minimisant ou évitant au maximum le phénomène de démélange.

Il s'ensuit que la solution apportée par la présente invention repose sur un procédé de fabrication d'un mélange gazeux contenant au moins un premier composé et au moins un deuxième composé dans des proportions prédéfinies, le premier composé étant choisi dans le groupe formé par O₂, N₂, He et N₂O, et le deuxième composé étant choisi dans le groupe formé par O₂, N₂He, N₂O et CO₂, dans lequel :
(a) on mélange en dynamique des proportions déterminées d'au moins le premier composé et le deuxième composé pour obtenir un mélange gazeux de composition désirée, ledit mélange en dynamique étant réalisé en introduisant de façon continue et/ou simultanée les constituants dudit mélange dans une chambre de mélange et/ou directement dans au moins un récipient de conditionnement, et dans la composition finale désirée,
(b) on ajuste la température dudit mélange gazeux contenant lesdits premier et deuxième composés obtenu à l'étape (a) pour la maintenir au-dessus de la température-seuil de démixtion dudit mélange.

Selon le cas, le procédé de fabrication de l'invention peut comprendre l'une ou plusieurs des caractéristiques suivantes :
- le deuxième composé est choisi parmi CO₂ et N₂O et la teneur en le deuxième composé (N₂O ou CO₂)est supérieure ou égale à 30% en volume, de préférence d'au moins 40% en volume.
- le premier composé est de l'oxygène et le deuxième composé est du protoxyde d'azote (N₂O), de préférence le mélange gazeux est constitué à 50% en vol. dudit premier composé et à 50% en vol. dudit deuxième composé, le premier composé étant de l'oxygène et le deuxième composé étant du protoxyde d'azote N₂O.
- l'un au moins desdits premier et deuxième composé est à l'état supercritique
- à l'étape (b), la température du mélange gazeux est ajustée ou maintenue supérieure à -5,5°C;
- la température du mélange gazeux est ajustée par réchauffage du mélange gazeux par échange thermique, de préférence ledit réchauffage est effectué avec au moins un réchauffeur électrique
- le mélange gazeux contient du CO₂ et O₂ et éventuellement de l'hélium
- la pression du mélange gazeux est comprise entre 120 bars et 300 bars,
- un premier composé et au moins un deuxième composé dans des proportions prédéfinies dans au moins un récipient, en particulier une bouteille de gaz, ledit mélange gazeux étant obtenu par un procédé de fabrication de mélange gazeux
- un mélange gazeux constitué à 50% en volume d'oxygène et à 50% en volume de protoxyde d'azote (N₂O), dans au moins un récipient, en particulier une bouteille de gaz, ledit mélange gazeux étant obtenu par un procédé de fabrication de mélange gazeux.

L'invention concerne aussi une installation de fabrication de mélanges gazeux en dynamique contenant au moins un premier composé et au moins un deuxième composé dans des proportions prédéfinies comportant :
- une source de premier composé contenant ledit premier composé,
- une source de deuxième composé contenant ledit deuxième composé,
- au moins une chambre de mélange en dynamique et des moyens pour introduire et mélanger des proportions déterminées, correspondant à un mélange gazeux de composition désirée, desdits premier et deuxième composés,
- des moyens d'ajustage de température situés en aval de ladite chambre de mélange permettant d'ajuster ou maintenir la température dudit mélange gazeux contenant lesdits premier et deuxième composés au-dessus de la température-seuil de démixtion dudit mélange gazeux.

De préférence, l'installation peut comprendre l'une ou l'autre des caractéristiques suivantes :
- les moyens d'ajustage de température sont choisis parmi les échangeurs thermiques, de préférence des réchauffeurs électriques.
- des moyens de compression situés en amont de ladite chambre, des moyens de réchauffage situés en amont de ladite chambre, et/ou au moins un réservoir tampon, de préférence l'installation comprend, agencés en série, les moyens de compressions, les moyens de réchauffage et le réservoir tampon.
- des moyens de commande agissant sur les moyens de compressions en réponse à une détection d'un seuil de pression prédéterminée par au moins un capteur de pression agencé de manière à pouvoir déterminer la pression régnant dans ledit réservoir tampon.

Selon encore un autre aspect, l'invention porte sur une installation de conditionnement de récipient de gaz, en particulier de bouteilles de gaz, comprenant :
- une installation de fabrication selon l'invention, et
- des moyens d'acheminement et de remplissage situés en aval des moyens d'ajustage de température et permettant de convoyer le mélange gazeux jusqu'à au moins un récipient de gaz à remplir avec ledit mélange gazeux et de remplir ledit récipient.

Dans le cadre de la présente invention, par mélange en dynamique, on entend un mélange réalisé en introduisant de façon continue et/ou simultanée les constituants dudit mélange dans une chambre de mélange et/ou directement dans des récipients de conditionnement, et ce, dans la composition finale attendue ou désirée.

En effet, les différents problèmes rencontrés avec les procédés de conditionnement classiques ne se posent pas avec un mélangeur dynamique selon l'invention et ce, pour plusieurs raisons et, tout d'abord, car le comptage des masses via un débitmètre massique permet de s'affranchir des incertitudes de mesures de température et de pression, et des aléas de fabrication liés aux imprécisions sur les quantités et valeurs mesurées qui existent avec lesdits procédés de conditionnement connus.

Le procédé de conditionnement de mélange gazeux "en dynamique" selon l'invention est particulièrement adapté à la réalisation et au conditionnement de mélanges gazeux destinés à une utilisation dans le domaine médical ou pharmaceutique, lesquels doivent répondre à des exigences sévères en termes de qualité et de précision de mélanges, notamment pour des questions évidentes de sécurité pour les patients.

La méthode de conditionnement en dynamique de l'invention est particulièrement adaptée à la fabrication de mélanges gazeux à base de O₂, N₂, He et N₂O, voire du CO₂ mais en une teneur volumique supérieure à 30 %.

Le procédé de conditionnement dynamique, encore appelé mélange dynamique, consiste à remplir, du début à la fin de la séquence de conditionnement, une ou des bouteilles de gaz avec un mélange gazeux ayant une composition finale souhaitée.

Le mélange des gaz est réalisé en amont de la rampe de conditionnement dans une chambre de mélange de très faible dimension où sont introduits les gaz entrant dans la composition du mélange à réaliser, les quantités introduites pour chaque gaz étant contrôlées par un débitmètre massique affecté à chaque source de chacun des constituants du mélange gazeux à réaliser.

Un ensemble de plusieurs vannes de régulations permet de contrôler le débit de gaz source grâce à l'action d'un système de régulation automatique.

De façon générale, le procédé de conditionnement en dynamique de l'invention présente les avantages principaux suivants :
- le mélange conditionné est homogène immédiatement, c'est-à-dire qu'il ne nécessite pas ensuite de roulage des bouteilles pour mélanger et bien homogénéiser les gaz qui s'y trouvent,
- il permet de limiter les écarts de réalisation de teneur pour un ensemble de bouteilles conditionnées lors d'un même cycle de conditionnement étant donné que le lot fabriqué peut être qualifié grâce à une seule analyse réalisée sur une bouteille du lot et on aboutit ainsi à un gain sur les coûts de contrôles analytiques et sur la durée de conditionnement,
- le comptage massique des quantités de gaz à conditionner présente l'intérêt de la précision de la mesure et donc de réaliser un comptage de quantité sur chaque gaz source indépendamment des conditions de pression et température. La précision du comptage massique permet alors d'obtenir un taux extrêmement faible de mélanges gazeux refusés après contrôle par analyse, et
- le fait de réaliser un comptage massique plutôt qu'une mesure des pressions et températures présente l'avantage d'éviter les imprécisions de mesure pouvant déboucher sur des mélanges gazeux et donc de résoudre le problème de l'obtention de mélanges gazeux dont les teneurs en les différents composés qui les composent sont non-conformes à celles souhaitées.

De plus, grâce au procédé de l'invention, les teneurs en les différents constituants dudit mélange gazeux au sein de plusieurs récipients conditionnés lors d'un même cycle de conditionnement sont reproductibles d'un récipient à l'autre, c'est-à-dire de bouteille de gaz à bouteille de gaz, puisque les écarts de fabrication sont extrêmement faibles d'une bouteille à l'autre. De là, un lot de plusieurs bouteilles remplies au cours d'un même cycle de conditionnement peut être contrôlé par analyse d'une seule bouteille prélevée au hasard dans le lot et il n'est plus nécessaire de répéter ce contrôle sur plusieurs bouteilles, comme cela est habituellement le cas, ce qui permet de gagner du temps et d'accroître la productivité et l'efficacité du procédé de conditionnement.

En d'autres termes, utiliser un mélangeur dynamique pour conditionner, en particulier des mélanges gazeux N₂O/O₂, présente un très bon facteur de reproductibilité du conditionnement dans le temps.

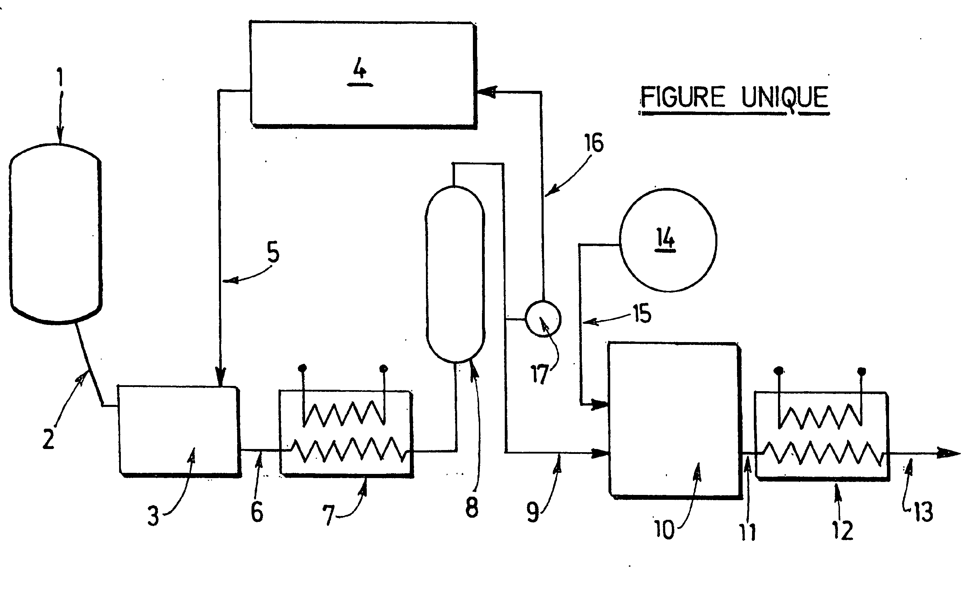

La présente invention va maintenant être mieux comprise grâce à la description détaillée suivante d'un mode de réalisation possible d'une installation de conditionnement en dynamique selon l'invention et de son fonctionnement, comme illustré sur la figure ci-jointe.

Une installation à mélangeur dynamique selon l'invention permettant la mise en condition supercritique du N₂O et son mélange subséquent avec de l'oxygène pour réaliser un mélange gazeux binaire 50% N₂O + 50% O₂ est détaillée sur la figure ci-jointe.

Cette installation comprend, en série, un réservoir 1 de N₂O stocké sous forme liquide, par exemple à une température d'environ -20°C et à une pression de l'ordre de 20 bars, alimentant par sa sortie et via la ligne 2 l'entrée d'une unité de compression 3 de gaz permettant de comprimer le N₂O liquide à une pression de 280 bars maximum et commandée (via 5) par une unité de commande 4 automatique du site.

Le fluide comprimé en 3 est acheminé par la ligne 6 jusqu'à un premier réchauffeur 7 fonctionnant à l'énergie électrique permettant de vaporiser et de réchauffer le N₂O à la température souhaitée, par exemple à la température souhaitée.

Un réservoir tampon 8 d'un volume de 500 litres (équivalent en eau) permet de stocker le N₂O gazeux chauffé, la température souhaitée, par exemple à 120°C environ à 120°C sortant du premier réchauffeur 7.

Un capteur de pression 17 permet de mesurer la pression de gaz dans le réservoir 8, ledit capteur 17 étant relié, via 16, à l'unité de commande 4 automatique de façon à rétroagir sur les moyens de compression 3 en fonction de la valeur de pression déterminée par le capteur 17.

Ainsi, lorsque la pression du gaz dans le réservoir 8 de gaz atteint 280 bars, alors l'unité 3 de compression et le réchauffeur 7 sont commandés à l'arrêt et l'unité de fabrication 10 de mélanges gazeux située en aval du réservoir 8 consomme le gaz stocké dans ledit réservoir 8.

A l'inverse, lorsque la pression du réservoir 8 de gaz descend en dessous d'une valeur minimale, par exemple 260 bars, l'unité de compression 3 et le réchauffeur 7 sont commandés à la marche jusqu'à ce le réservoir 8 atteigne à nouveau 280 bars.

Le réservoir 8 de N₂O gazeux alimente l'unité de fabrication de mélange en dynamique 10 via la ligne 9.

Le mélangeur dynamique 10 reçoit alors du gaz à une pression moyenne de l'ordre de 270 bar de N₂O à une température d'environ 120°C, d'une part, et de l'oxygène gazeux à température ambiante et à une pression moyenne de l'ordre de 270 bars issu d'une source 14 d'oxygène gazeux et amené par une ligne 15, d'autre part.

Le mélange gazeux aux proportions souhaitées de N₂O et de O2 est alors obtenu, par exemple un mélange 50%/50%.

La sortie de l'unité de fabrication de mélange en dynamique 10 est, conformément à la présente invention, reliée par une ligne 11 à un second réchauffeur 12 électrique destiné à maintenir le mélange gazeux réalisé dans l'unité 10 à une température supérieure à son point de démélange.

Ensuite, le mélange gazeux est envoyé, via la ligne 13, vers un ou des récipients de gaz (non montrés) qui sont remplis avec le mélange gazeux 50%/50% en O₂/N₂O ainsi réalisé.

Dans ce cas, le réservoir 1 de N₂O sous forme liquide a été choisi du fait de l'intérêt du mode de stockage liquide du N₂O dans le cas des consommations importantes.

Cependant, il va de soi que des bouteilles ou d'autres sources de N₂O sous forme de gaz pourraient aussi être utilisées dans le cas de consommations plus faibles et l'unité de compression 3 comprimerait alors du N₂O gazeux et le réchauffeur 7 électrique aurait pour fonction unique de réchauffer ledit N₂O.

De façon analogue, la source d'oxygène 14 contient de l'oxygène sous forme gazeuse mais on peut imaginer utiliser de l'oxygène liquide si les consommations le justifient et, dans ce cas, il est possible de prévoir un troisième réchauffeur sur la ligne 15 de manière à vaporiser et réchauffer l'oxygène liquide jusqu'à la température souhaitée, à savoir la température ambiante (1°C à 45°C environ).

Par ailleurs, le choix de l'énergie électrique pour l'alimentation des réchauffeurs 3 et 12 est directement lié aux sources d'énergie disponibles sur le site de l'application. Toutefois, là encore, d'autres sources d'énergie peuvent être utilisées, telles que vapeur, sources de chaleur provenant d'unités ou de procédés dégageant de la chaleur ou nécessitant un refroidissement.

Le conditionnement de récipients à des pressions inférieures à 200 bars pourrait, en outre, conduire à définir des pressions différentes au sein du système, notamment des pressions dans les lignes inférieures, par exemple des pressions de conditionnement de 150 ou 170 bars.

Au vu de ce qui précède, on comprend que le premier aspect de l'invention concerne la mise en condition supercritique du gaz, ici le N₂O, afin de conditionner le mélange gazeux en dynamique.

Le second aspect de l'invention concerne l'amélioration du conditionnement en dynamique des mélanges ayant une teneur de plus de 30% en gaz liquéfié, tel que le mélange gazeux analgésique 50% O₂ + 50% N₂O susmentionné.

Au cours du conditionnement des récipients, en l'absence des moyens de réchauffage de gaz 12 situé en aval de la chambre de mélange 10, du fait de la détente des gaz en aval de ladite chambre 10 de mélange, les mélanges gazeux se refroidissent et la température atteinte peut être inférieure à la température de démélange du produit à conditionner, ce qui dégrade la reproductibilité de réalisation des mélanges dans les différentes bouteilles conditionnée d'une même rampe.

Or, conformément à l'invention, en utilisant un réchauffeur 12 en aval de ladite chambre 10 de mélange gazeux, comme expliqué ci-dessus, on évite ce problème car la température est maintenue en permanence au-dessus de la température de démélange du produit gazeux à conditionner.

L'invention repose en fait sur une utilisation judicieuse du comportement à l'état supercritique du N₂O en vue de conditionner des mélanges gazeux notamment médicaux ou à usage médical.

En effet, le N₂O est un gaz liquéfié devant également être mis à l'état supercritique pour réaliser en dynamique des mélanges gazeux fiables et conformes au but visé, à savoir teneurs conformes et précision élevée.

Les mécanismes fondamentaux concernant l'état supercritique du N₂O sont peu connus à ce jour et ne font pas partie de la présente invention. En effet, les courbes d'enthalpie, d'entropie, de pression et de température du N₂O ne font pas état des conditions supercritiques. En outre, la littérature scientifique ne fait non plus état de la stabilité de la molécule de N₂O et de la non-dégradation du N₂O en NO, NOx, O₂, dans ces conditions de pression et de température, notamment environ 270 bars et environ 120°C.

Il est à souligner qu'une pression inférieure aurait pu être déterminée pour le N₂O supercritique car la valeur retenue de 270 bars est liée à la pression de la source de O₂ qui doit permettre le conditionnement de mélanges à environ 200 bars.

L'unité de fabrication dynamique de l'invention fabriquer des mélanges de différentes compositions à partir de plusieurs sources de gaz simples et à différentes pressions de conditionnement finales des récipients. De préférence, les pressions des différentes sources de gaz sont alignées sur la pression maximum nécessaire, c'est-à-dire par exemple 270 bars.

Afin de vérifier la non-dégradation du N₂O à l'état supercritique, des tests ont été menés et ont montré que les taux de NO et NOx retrouvés dans les échantillons sont inférieurs aux seuils définis dans les spécifications définies par la Pharmacopée du N₂O et sont également inférieurs aux taux garantis par les spécifications de base du N₂O en vrac en sortie d'usine, c'est-à-dire une teneur inférieure ou égale à 2 ppm en volume de NO.

Grâce à l'invention, le problème de démélange du mélange gazeux est résolu de par la mise en oeuvre du dispositif de réchauffage 12 des gaz en sortie de la chambre 10 de mélange pour maintenir le mélange gazeux dans les conditions de température supérieures à la température de démélange durant le cycle de remplissage des emballages. Le mélange étant ainsi maintenu à l'état gazeux, l'homogénéité du mélange est conservée et les écarts de teneurs du mélange réalisés sont suffisants faibles pour permettre de contrôler un lot de bouteilles par analyse d'une seule bouteille prélevée dans la rampe de conditionnement.

Il est à souligner que, selon l'art antérieur, le conditionnement de mélanges gazeux contenant moins de 30% de CO₂ n'a jamais mis en évidence ce problème de démélange après la chambre de détente 10 du mélangeur car la température de démélange d'un gaz contenant moins de 30% de CO₂ se situe à environ -30°C. Or, cette température n'est atteinte en sortie de la chambre 10 de mélange que pendant une courte période lors de la phase de conditionnement.

## Revendications

1. Procédé de fabrication d'un mélange gazeux contenant au moins un premier composé et au moins un deuxième composé dans des proportions prédéfinies, le premier composé étant choisi dans le groupe formé par O₂, N₂, He et N₂O, et le deuxième composé étant choisi dans le groupe formé par O₂, N₂, He, N₂O et CO₂, dans lequel
(a) on mélange en dynamique des proportions déterminées d'au moins le premier composé et le deuxième composé pour obtenir un mélange gazeux de composition désirée, ledit mélange en dynamique étant réalisé en introduisant de façon continue et/ou simultanée les constituants dudit mélange dans une chambre de mélange et/ou directement dans au moins un récipient de conditionnement, et dans la composition finale désirée,
(b) on ajuste la température dudit mélange gazeux contenant lesdits premier et deuxième composés obtenu à l'étape (a) pour la maintenir au-dessus de la température-seuil de démixtion dudit mélange.

2. Procédé selon la revendication 1, **caractérisé en ce que** le deuxième composé est choisi parmi CO₂ et N₂O et la teneur du deuxième composé N₂O ou CO₂ est supérieure ou égale à 30 % en volume, de préférence d'au moins 40 % en volume.

3. Procédé selon l'une des revendications 1 ou 2, **caractérisé en ce que** le premier composé est de l'oxygène et le deuxième composé est du protoxyde d'azote (N₂O), de préférence le mélange gazeux est constitué à 50% en vol. dudit premier composé et à 50% en vol. dudit deuxième composé, le premier composé étant de l'oxygène et le deuxième composé étant du protoxyde d'azote (N₂O).

4. Procédé selon l'une des revendications 1 à 3, **caractérisé en ce que** l'un au moins desdits premier et deuxième composés est à l'état supercritique.

5. Procédé selon l'une des revendications 1 à 4, **caractérisé en ce qu'**à l'étape (b), la température du mélange gazeux est ajustée ou maintenue supérieure à -5,5°C.

6. Procédé selon l'une des revendications 1 à 5, **caractérisé en ce que** la température du mélange gazeux est ajustée par réchauffage du mélange gazeux par échange thermique, de préférence ledit réchauffage est effectué avec au moins un réchauffeur électrique.

7. Procédé selon la revendication 1, **caractérisé en ce que** le mélange gazeux contient du CO₂ et du O₂, et éventuellement de l'hélium.

8. Procédé selon l'une des revendications 1 à 7, **caractérisé en ce que** la pression du mélange gazeux est comprise entre 120 bars et 300 bars.

9. Procédé de conditionnement de récipient de gaz dans lequel on introduit un mélange gazeux contenant au moins un premier composé et au moins un deuxième composé dans des proportions prédéfinies dans au moins un récipient, en particulier une bouteille de gaz, ledit mélange gazeux étant obtenu par un procédé de fabrication de mélange gazeux selon l'une des revendications 1 à 8.

10. Procédé selon la revendication 9, **caractérisé en ce que** le mélange gazeux est constitué à 50% en volume d'oxygène et à 50% en volume de protoxyde d' azote (N₂O).

11. Installation de fabrication d'un mélange gazeux en dynamique contenant au moins un premier composé et au moins un deuxième composé dans des proportions prédéfinies, comportant :
- une source de premier composé (14) contenant ledit premier composé,
- une source de deuxième composé (1) contenant ledit deuxième composé,
- au moins une chambre de mélange (10) en dynamique et des moyens pour introduire et mélanger des proportions déterminées, correspondant à un mélange gazeux de composition désirée, desdits premier et deuxième composés,
- des moyens d'ajustage (12) de température situés en aval de ladite chambre de mélange (10) permettant d'ajuster ou maintenir la température dudit mélange gazeux contenant lesdits premier et deuxième composés au-dessus de la température-seuil de démixtion dudit mélange gazeux.

12. Installation de conditionnement de récipient de gaz, en particulier de bouteilles de gaz, comprenant :
- une installation de fabrication selon la revendication 11, et
- des moyens d'acheminement et de remplissage (13) situés en aval des moyens d'ajustage (12) de température et permettant de convoyer le mélange gazeux jusqu'à au moins un récipient de gaz à remplir avec ledit mélange gazeux et de remplir ledit récipient.

## Patentansprüche

1. Verfahren zur Herstellung eines gasförmigen Gemischs, welches mindestens eine erste Verbindung und mindestens eine zweite Verbindung in vorbestimmten Anteilen enthält, wobei die erste Verbindung ausgewählt ist aus der Gruppe bestehend aus O₂, N₂, He und N₂O und die zweite Verbindung ausgewählt ist aus der Gruppe bestehend aus O₂, N₂, He, N₂O und CO₂, bei dem:
(a) bestimmte Anteile mindestens der ersten Verbindung und der zweiten Verbindung dynamisch gemischt werden, um ein gewünschtes gasförmiges Gemisch zu erhalten, wobei das dynamische Mischen durch Einleiten der Bestandteile des Gemischs kontinuierlich und/oder gleichzeitig in eine Mischkammer und/oder direkt in mindestens einen Abfüllbehälter zustande kommt, und bei der gewünschten Endzusammensetzung
(b) die Temperatur der in Schritt (a) erhaltenen Gasmischung, welche die ersten und zweiten Verbindungen enthält, eingestellt wird, um diese oberhalb der Schwellentemperatur der Entmischung des Gemischs aufrecht zu erhalten.

2. Verfahren nach Anspruch 1, **dadurch gekennzeichnet, dass** die zweite Verbindung ausgewählt ist aus CO₂ und N₂O und der Gehalt der zweiten Verbindung N₂O oder CO₂ mehr als oder genau 30 Volumenprozent, vorzugsweise mindestens 40 Volumenprozent beträgt.

3. Verfahren nach einem der Ansprüche 1 oder 2, **dadurch gekennzeichnet, dass** die erste Verbindung Sauerstoff und die zweite Verbindung Distickstoffmonoxid (N₂O) ist, vorzugsweise besteht das gasförmige Gemisch aus 50 Vol.% der ersten Verbindung und aus 50 Vol.% der zweiten Verbindung, wobei die erste Verbindung Sauerstoff und die zweite Verbindung Distickstoffmonoxid (N₂O) ist.

4. Verfahren nach einem der Ansprüche 1 bis 3, **dadurch gekennzeichnet, dass** sich mindestens eine der ersten und zweiten Verbindungen in hoch kritischem Zustand befindet.

5. Verfahren nach einem der Ansprüche 1 bis 4, **dadurch gekennzeichnet, dass** im Schritt (b) die Temperatur des gasförmigen Gemischs oberhalb von -5,5 °C eingestellt oder aufrechterhalten wird.

6. Verfahren nach einem der Ansprüche 1 bis 5, **dadurch gekennzeichnet, dass** die Temperatur des gasförmigen Gemischs durch Erwärmung des gasförmigen Gemischs mittels Wärmeaustausch eingestellt wird, wobei, die Erwärmung vorzugsweise mit mindestens einem Elektroerhitzer durchgeführt wird.

7. Verfahren nach Anspruch 1, **dadurch gekennzeichnet, dass** das gasförmige Gemisch CO₂ und O₂ sowie gegebenenfalls Helium enthält.

8. Verfahren nach einem der Ansprüche 1 bis 7, **dadurch gekennzeichnet, dass** der Druck des gasförmigen Gemischs zwischen 120 Bar und 300 Bar liegt.

9. Verfahren zum Abfüllen eines Gasbehälters, bei dem ein gasförmiges Gemisch, welches mindestens eine erste Verbindung und mindestens eine zweite Verbindung in vorbestimmten Anteilen enthält, in mindestens einen Behälter, insbesondere eine Gasflasche eingeleitet wird, wobei das gasförmige Gemisch durch ein Verfahren zur Herstellung eines gasförmigen Gemischs nach einem der Ansprüche 1 bis 8 erhalten wird.

10. Verfahren nach Anspruch 9, **dadurch gekennzeichnet, dass** das gasförmige Gemisch aus 50 Volumenprozent Sauerstoff und 50 Volumenprozent Distickstoffmonoxid (N₂O) besteht.

11. Einrichtung zur dynamischen Herstellung eines gasförmigen Gemischs, welches mindestens eine erste Verbindung und mindestens eine zweite Verbindung in vorbestimmten Anteilen enthält, umfassend:
- eine Quelle der ersten Verbindung (14), welche die erste Verbindung enthält,
- eine Quelle der zweiten Verbindung (1), welche die zweite Verbindung enthält,
- mindestens eine dynamische Mischkammer (10) und Mittel zum Einleiten und Mischen bestimmter Anteile der ersten und zweiten Verbindungen, die einem gasförmigen Gemisch der gewünschten Zusammensetzung entsprechen,
- Mittel zum Einstellen der Temperatur (12), die sich hinter der Mischkammer (10) befinden, welche das Einstellen oder Aufrechterhalten der Temperatur des gasförmigen Gemischs, das die ersten und zweiten Verbindungen enthält, oberhalb der Schwellentemperatur der Entmischung des gasförmigen Gemischs erlauben.

12. Einrichtung zum Abfüllen eines Gasbehälters, insbesondere von Gasflaschen, umfassend:
- eine Einrichtung zur Herstellung nach Anspruch 11 sowie
- Mittel zum Leiten und Abfüllen (13), die sich hinter den Mitteln zum Einstellen der Temperatur (12) befinden und die es ermöglichen, das gasförmige Gemisch zu mindestens einem mit dem gasförmigen Gemisch zu befüllenden Gasbehälter zu befördern und den Behälter zu füllen.

## Claims

1. Process for manufacturing a gas mixture containing at least a first component and at least a second component in predefined proportions, the first component being chosen from the group formed by O₂, N₂, He and N₂O, and the second component being chosen from the group formed by O₂, N₂, He, N₂O and CO₂, in which:
(a) predetermined proportions of at least the first component and the second component are mixed dynamically in order to obtain a gas mixture of the desired composition, the said dynamic mixing being carried out by continuously and/or simultaneously introducing the constituents of the said mixture into a mixing chamber and/or directly into at least one container, and in the desired final composition; and
(b) the temperature of the said gas mixture containing the said first and second components obtained in step (a) is adjusted in order to keep it above the demixing threshold temperature of the said mixture.

2. Process according to Claim 1, **characterized in that** the second component is chosen from CO₂ and N₂O and the content of the second component N₂O or CO₂ is greater than or equal to 30 vol%, preferably at least 40 vol%.

3. Process according to either of Claims 1 and 2, **characterized in that** the first component is oxygen and the second component is nitrous oxide (N₂O) and preferably the gas mixture consists of 50 vol% of the said first component and 50 vol% of the said second component, the first component being oxygen and the second component being nitrous oxide (N₂O).

4. Process according to one of Claims 1 to 3, **characterized in that** at least one of the said first and second components is in the supercritical state.

5. Process according to one of Claims 1 to 4, **characterized in that** in step (b), the temperature of the gas mixture is adjusted to or kept above -5.5°C.

6. Process according to one of Claims 1 to 5, **characterized in that** the temperature of the gas mixture is adjusted by warming the gas mixture by heat exchange, preferably the said warming being carried out by at least one electric heater.

7. Process according to Claim 1, **characterized in that** the gas mixture contains CO₂ and O₂, and possibly helium.

8. Process according to one of Claims 1 to 7, **characterized in that** the pressure of the gas mixture is between 120 bar and 300 bar.

9. Process for filling a container with gas, in which a gas mixture containing at least a first component and at least a second component in predefined proportions is injected into at least one container, particularly a gas cylinder, the said gas mixture being obtained by a gas mixture manufacturing process according to one of Claims 1 to 8.

10. Process according to Claim 9, **characterized in that** the gas mixture consists of 50 vol% oxygen and 50 vol% nitrous oxide (N₂O).

11. Plant for manufacturing gas mixtures dynamically, these containing at least a first component and at least a second component in predefined proportions, which plant comprises:
- a source (14) of the first component, containing the said first component;
- a source (1) of the second component, containing the said second component;
- at least one dynamic mixing chamber (10) and means for introducing and mixing specified proportions, corresponding to a gas mixture of desired composition, of the said first and second components; and
- temperature adjustment means (12) located downstream of the said mixing chamber (10) allowing the temperature of the said gas mixture containing the said first and second components to be adjusted or kept above the demixing threshold temperature of the said gas mixture.

12. Plant for filling containers with gas, particularly gas cylinders, which comprises:
- a manufacturing plant according to Claim 11; and
- conveying and filling means (13) located downstream of the temperature adjustment means (12) and making it possible to convey the gas mixture to at least one gas container to be filled with the said gas mixture and to fill the said container.
